(19) **Europäisches Patentamt European Patent Office Office européen des brevets**

(11) **EP 3 651 159 A1**

(12) **EUROPEAN PATENT APPLICATION**

(43) Date of publication:
**13.05.2020 Bulletin 2020/20**

(21) Application number: **18205425.4**

(22) Date of filing: **09.11.2018**

(51) Int Cl.:
**G16H 40/20** (2018.01)  **G16H 50/70** (2018.01)
**G06N 5/02** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **WU, Qifan**
**5656 AE Eindhoven (NL)**

• **JIANG, Ze Yu**
**5656 AE Eindhoven (NL)**
• **CHAN, Cyrus**
**5656 AE Eindhoven (NL)**
• **CHIAU, Choo Chiap**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **METHOD AND SYSTEM FOR ANALYZING DATA IN THE HEALTHCARE DOMAIN**

(57)    A method (12) for analyzing data in the healthcare domain in order to determine groups of factors in a dataset whose co-occurrence leads to the satisfying of at least one condition the healthcare domain, the factors and the condition satisfaction value being fields within said dataset. An adaptation of the Apriori Algorithm is applied whereby, for each of a plurality of different sets of factors in the dataset, Confidence and/or Lift values are calculated (18) in respect of co-occurrence of the factors of the subset and positive satisfaction of the condition. Based on these Confidence and/or Lift values for the multiple different sets of factors, it is determined (20) which of the factors of the dataset together can be concluded to lead to a Yes value for the condition result.

FIG. 1

**Description**

FIELD OF THE INVENTION

The invention relates to a method and system for analyzing data in the healthcare domain, and more particularly for determining factors in a healthcare dataset which lead to the satisfying of one or more conditions included in the dataset.

BACKGROUND OF THE INVENTION

Recent rapid advancement in Healthcare IT (HIT) has led to large volumes of clinical data and information being available in most hospitals and medical centers. This data pertains not only to a medical record and history of patients but also to data pertaining to the running and functioning of the hospital, such as patient waiting times and 5-year postoperative survival rates for instance.

It is common for medical centers and hospitals to set key performance indicators (KPIs) which are monitored and tracked to check that a hospital is meeting key requirements in certain areas. These may include for example success in patient outcomes, thresholds in patient satisfaction ratings, maximum allowable rates for infection contraction, waiting time thresholds, mortality rates in certain domains, average length of patient stay for certain treatments or clinical domains, and others.

Satisfying of the key performance indicators may involve the satisfying of one or more subsidiary conditions.

To assist in meeting the key conditions, it is valuable to analyze patient data to identify any factors or attributes which are linked or associated with positive satisfaction values for the various conditions.

One approach for achieving this is to determine a level of association between each of a plurality of factors and the satisfying of the condition, the factors being included as fields in a patient dataset, and then ranking each of the factors according to its level of association. Those near the top of the ranking list are then identified or selected as being important factors to focus on for improving satisfaction of the condition(s).

However, this approach cannot take into account the linkage and interdependency that exists between certain factors.

An improved approach to analyzing association between medical factors or attributes and the satisfying of one or more conditions in the healthcare domain is sought.

SUMMARY OF THE INVENTION

The invention is defined by the claims.

According to examples in accordance with an aspect of the invention, there is provided a method for determining factors in a dataset whose co-occurrence lead to satisfying of at least one condition in the healthcare domain, the method comprising:

retrieving a dataset, P, the fields of the dataset including a plurality of healthcare factors, and at least one condition result in the healthcare domain, wherein each of the factors and the condition result have binary, Yes/No, values;

calculating, for each of a plurality of plural subsets, (A, B,....,n), of the factors, Confidence, and/or Lift values using the Apriori Algorithm, these having the form,

$$\text{confidence}(X \cup Y) = \frac{support(X \cup Y)}{support(X)}$$

$$\text{lift}(X \to Y) = \frac{support(X \cup Y)}{support(X) * support(Y)}$$

wherein co-occurrence in a single record of Yes values for each of the factors of a given subset is used as X in the Algorithm, and a Yes value of the condition result in said same record is used as Y in the Algorithm, and where, in general for a given factor or condition $\phi$,

$$support(\phi) = \frac{|\phi|}{|P|}$$

and;
determining factors of the dataset which together lead to a Yes value for the condition result based on the calculated Confidence and/or Lift values for the plurality of subsets of factors.

The factors determined as together leading to a Yes value for the condition result may be selected from the plurality of subsets of factors (for which confidence and/or lift values are calculated).

Embodiments of the invention involve determining particular factors of the dataset which together reliably lead to a positive (Yes) value for the condition result. This may mean determining those factors whose co-occurrence leads to a positive (Yes) value for the condition result.

This is achieved by determining confidence and/or lift values for each of a plurality of subsets of factors in the dataset, each subset including a plurality of the factors. In this way, confidence and/or lift values are determined in respect of the co-occurrence of multiple different factors, and furthermore in respect of plural different groups or subsets of multiple factors (e.g. with different numbers and combinations of factors). The confidence and/or lift values give an indication as to which of these subsets of plural factors together reliably lead a positive (Yes) value for the condition. By testing different subsets of factors, preferably with different numbers and combinations of factors, it can be determined a group of the factors whose co-occurrence most reliably leads to a positive (Yes) value for the condition result.

Applied in the healthcare domain, this can lead to improvement in patient outcomes within hospitals and medical centers, since linkage between groups of factors associated with individual patients, such as factors related to medical status or history, or behavior or practices of the patient, and conditions e.g. related to patient or treatment outcome or other requirements related to treatment or consultation, e.g. waiting time, can be determined. This allows the conditions to be more reliably met in future, since the combinations of factors leading to those conditions, or failure in those conditions, can be identified.

The invention is based on application of an adaptation of the Apriori Algorithm. This algorithm is a well-known algorithm within the field of machine learning, and is used for frequent item set mining and association rule learning over databases of interrelated factors. Frequent item sets identified through the Apriori Algorithm can be used to determine association rules which highlight general trends in the database. By association rules is meant relations between variables in a database. In the present case, an adaptation of the algorithm is applied to identify association rules between each of a plurality of plural sets or groups of factors (fields) in a dataset and the satisfying of some condition, also listed as a field in the database. By this is meant that the algorithm is applied to seek plural sets or groups of factors which, based on the records in the dataset, tend to lead reliably to a positive (Yes) value for the condition result. This will be explained in greater detail below.

Embodiments of the invention take into account that it is often not a single factor which leads to a particular condition result, but rather the interaction and combination of multiple factors. Prior art approaches in general are based on ranking single factors in terms of their frequency of association with a given condition result. The highest ranking factors are then selected as those which lead to the condition result, and hence those which should be focused on for improvement in frequency of attaining the condition result.

However, this can give a skewed result, because some factors may be ordered relatively low in such a ranking, but which, when present in combination with certain other factors, lead to a very high likelihood of the condition being satisfied. It is of value to identify such factors since by focusing on these, in combination with those others, the condition might more reliably be satisfied than by focusing on the high ranking factors alone.

Equivalently, in certain cases it may be the aim to avoid the satisfying of a certain condition. Here again it of value to identify factors which in combination with others very reliably lead to satisfying of the condition. By avoiding or improving just one of those identified factors (e.g. one which is easiest to avoid or improve), satisfying of the condition in a significant number of cases might be avoided.

For the avoidance of doubt, 'plural subsets' refers to subsets including plural factors.

Following retrieval of the dataset, P, the method may optionally comprise a pre-filtering step or procedure adapted to reduce a number of records in the dataset having a negative (No) value for the condition result. In particular, the pre-filtering step may be adapted to reduce the number of records having a negative (No) value more than (or to a greater extent than) it reduces the number of records having a positive (Yes) value. The pre-filtering step may have a bias in favor of records having a positive (Yes) value for the condition result.

Since the method aims at identifying associations between groups of factors and positive (Yes) values for the condition result, records containing a negative result are less relevant or useful to the method. Removing at least a portion of these before performing further processing reduces the quantity of data which must be processed (e.g. for which confidence and/or lift values must be determined), and hence improves processing efficiency and speed.

According to one or more embodiments, the condition may be a Performance Indicator for a healthcare provider to which data in the dataset relates.

Performance indicators, or Key Performance Indicators (KPIs), are frequently set by hospital and healthcare centers, and monitored and tracked to check that a hospital is meeting key requirements in certain areas. These may include for example success in patient outcomes, thresholds in patient satisfaction ratings, maximum allowable rates for infection contraction, waiting time thresholds, mortality rates in certain domains, average length of patient stay for certain treatments or clinical domains and many others.

The fields of the dataset may encompass a plurality of condition results in the healthcare domain. The method may involve performing each of said steps of: calculating confidence and/or lift values, and determining which of the plurality of subsets of factors leads to a positive (Yes) value for the condition result, in respect of each of the plurality of condition results. In the case that the method includes the optional pre-filtering step, performed following retrieval of the dataset, P, and in advance of calculating the confidence and/or lift values, then said pre-filtering step may also be performed in respect of each of said plurality of condition results.

In this way associations between subsets of factors in the dataset and multiple different conditions can be separately identified.

The method may according to one or more embodiments be for determining factors in the dataset whose co-occurrence leads to satisfying of a performance Target in a healthcare domain, satisfying of said Target involving satisfying of said plurality of condition results referred to above. The Target may be a performance indicator, e.g. a key performance indicator (KPI).

Once subsets of factors have been individually identified which lead to each of the plural conditions encompassed by the Target, the factors of these subsets may together be determined as those that lead to satisfying of the Target. These may be output as a data output for example.

In advantageous examples, those factors which are found to contribute to the satisfying of each and every one of the plural conditions may be together determined as those that lead to satisfying of the Target. These may be output as a data output for example.

According to one or more embodiments, the plurality of plural subsets, (A, B,...,n), of the factors may comprise subsets containing different number of factors. By this is meant that certain of the subsets contain different numbers of factors to other of the subsets. This means that the method is not limited by any pre-prepared assumption concerning how many factors tend to lead to a particular condition result. The method can determine both how many factors, and which factors, lead to a certain condition result.

As noted above, the method involves determining confidence and/or lift values of the Apriori Algorithm in respect of each of the plurality of subsets of factors. These values may be determined sequentially, with the outcome for one (e.g. in respect of all of the subsets) informing whether the other is to be determined.

This is can reduce processing demand and increase processing speed since in at least some cases, only the confidence values need to be determined and not the lift values, or vice versa.

By way of example, in accordance with one or more sets of embodiments, the method may comprise:

first calculating Confidence values in respect of each of said plurality of subsets of factors,
identifying a maximum of the calculated Confidence values; and
determining Lift values only in the case that more than one of said subsets shares said maximum confidence value, the lift values being determined only in respect of said subsets sharing the maximum confidence value.

Hence here, a maximum, or largest, of the calculated confidence values is identified. Only if this maximum value has been calculated for more than one of the plurality of subsets of factors are Lift values then calculated, these being calculated only for those subsets for which the maximum Confidence value was calculated. The Lift values are hence used to narrow down the subsets, such that those subsets with the greatest association with the condition result can be identified.

By contrast, in the case that said maximum of the confidence values is returned for only one of said subsets of factors, the factors of that subset may be determined as the factors which lead to a positive (Yes) value of said condition result.

Hence, in this case, no Lift values are determined, and the output of the method is the single subset of factors returning the highest confidence value.

In the case that said Lift values are determined however, according to particular examples, the method may further comprise (after determining the lift values in respect of the subsets of factors sharing the maximum confidence value):

identifying a maximum of the calculated Lift values;
identifying which of the subsets of factors said maximum Lift value has been calculated for; and
determining the factors of said identified subset(s) to be the factors which lead to a positive (Yes) value of said condition result.

Hence in this case, the factors belonging to any and all subsets sharing the maximum returned Lift value are determined as the factors which lead to a positive (Yes) value of said condition result, i.e. the output of the method.

According to one or more examples, the method may further comprise applying a minimum threshold to the initially calculated confidence values, such that a maximum is identified only among the calculated confidence values exceeding said threshold. This effectively filters the confidence values, to reduce the total quantity of results which need to be compared in order to determine the maximum value.

According to one or more examples, the at least one condition may include the satisfying of a minimum survival period of a patient following a medical procedure, for example a 5-year survival period. This is a very common performance indicator used in hospitals following surgery or some other medical intervention.

This is an example of a condition relating to patient outcome or to treatment success.

According to one or more examples, the at least one condition may include compliance with a maximum patient waiting time threshold.

This is an example of a condition relating to the running and organization of a hospital or medical center, or to patient experience at the hospital or medical center.

Examples according to a further aspect of the invention provide a computer program product comprising a non-transitory computer readable medium, the computer readable medium having computer readable code embodied therein, the computer readable code being configured such that, on execution by a suitable computer or processor, the computer or processor is caused to performed a method in accordance with any of the examples or embodiments outlined above or described below, or in accordance with any claim of this application.

Examples according to a further aspect of the present invention provide a system comprising:

a memory comprising instruction data representing a set of instructions; and
a processor configured to communicate with the memory and to execute the set of instructions, wherein the set of instructions, when executed by the processor, cause the processor to:

retrieve a dataset, P, the fields of the dataset including a plurality of healthcare factors, and at least one condition result in the healthcare domain, wherein each of the factors and the condition result have binary values;
calculate, for each of a plurality of plural subsets, (A, B,....,n), of the factors, Confidence, and/or Lift values using the Apriori Algorithm, these having the form,

$$\text{confidence}(X \cup Y) = \frac{support(X \cup Y)}{support(X)}$$

$$\text{lift}(X \rightarrow Y) = \frac{support(X \cup Y)}{support(X) * support(Y)}$$

wherein co-occurrence of Yes values for each of the factors in a given subset is used as X in the Algorithm, and a Yes value of the condition result is used as Y in the Algorithm, and where, for a given factor or condition $\phi$,

$$support(\phi) = \frac{|\phi|}{|P|}$$

and;
determine which of the factors of the dataset lead to a Yes value for the condition result based on the calculated Confidence and/or Lift values for the plurality of subsets of the factors.

Any embodiment, example or variation described above in relation to the method aspect of this invention may be applied with equal advantage to the presently described system aspect of the invention.

According to one or more sets of embodiments for instance, said set of instructions, when executed by the processor, may cause the processor to

first calculate Confidence values in respect of each of said plurality of subsets of factors,

identify a maximum of the calculated Confidence values; and

determine Lift values only in the case that more than one of said subsets shares said maximum confidence value, the lift values being determined only in respect of said subsets sharing the maximum confidence value.

According to certain examples, in the case that said maximum of the confidence values is returned for only one of said subsets of factors, the factors of that subset may be determined to be the factors which lead to a Yes value of said condition result.

According to certain examples, in the case that said Lift values are determined, the instructions, when executed by the processor, further cause the processor to:

identify a maximum of the calculated Lift values;

identify which of the subsets of factors said maximum Lift value has been calculated for; and

determine the factors of said identified subset(s) to be the factors which lead to a Yes value of said condition result.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 shows a block diagram of an example method in accordance with one or more embodiments;

Fig. 2 shows a block diagram of a further example method in accordance with one or more embodiments; and

Fig. 3 schematically depicts in block diagram form an example system in accordance with one or more embodiments.

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method for analyzing data in the healthcare domain in order to determine groups of factors in a dataset whose co-occurrence leads to the satisfying of at least one condition the healthcare domain, the factors and the condition satisfaction value being fields within said dataset. An adaptation of the Apriori Algorithm is applied whereby, for each of a plurality of different sets of factors in the dataset, Confidence and/or Lift values are calculated in respect of co-occurrence of the factors of the subset and positive satisfaction of the condition. Based on these Confidence and/or Lift values for the multiple different sets of factors, it is determined which of the factors of the dataset together can be concluded to lead to a Yes value for the condition result.

In certain fields outside of the healthcare domain, data analytics and machine learning techniques are known and are many be applied on a regular basis, for instance daily, and in some cases are used for real-time decision making. These techniques and approaches have been applied for instance in fields such as retail, e-commerce, marketing, and banking.

The models that are utilized in these approaches are mathematical or statistical models, which, by themselves, are neutral or independent to the fields to which they are applied. Therefore, it has been realised by the inventors that some of the machine learning algorithms or models might be advantageously applied to data analytics in the healthcare domain, with certain modifications being made. The benefit of such tools is the facilitation of faster and more intelligent analysis of very large quantities of clinical and medical data, this data already being stored in existing Healthcare IT systems, to permit trends to be identified. This can permit practices to be adapted to allow certain desired outcomes (such as satisfying certain conditions or targets) to be more reliably attained.

The present invention is based on an adaptation of a well-known machine learning algorithm, the Apriori Algorithm, or, more broadly, Association Rules Mining algorithm. This development adapts this algorithm or algorithmic approach more specifically to the healthcare domain to help resolve the disadvantages described in previous sections pertaining to known analysis methods in the healthcare domain.

The functioning of the Apriori or Association Rules algorithm in general will now be described.

In general, the Apriori algorithm and Association Rules Mining make use of three specific parameters which are used to identify a degree of association between two items, X and Y, in a dataset P. More particularly, the aim is generally to identify the extent to which the presence of one of these items, e.g. X, implies or entails presence of the other. This can be expressed in terms of a rule: $X \rightarrow Y$. These specific parameters are known as: Support, Confidence and Lift.

.Given X and Y as two items in a dataset P, with the relevant rule consequently being $X \rightarrow Y$, Support, Confidence and Lift may be understood as follows.

Support: an indication of how frequently the chosen set (X, Y) appears in the dataset, i.e. how frequently items X and Y are found together in a single record of the database. This can be defined more precisely by the expression:

$$support(X \cup Y) = \frac{|X \cup Y|}{|P|}$$

Confidence: an indication of how often the rule $X \rightarrow Y$ has been found to be true, i.e. in how many cases of X appearing in a record, Y also appears in the same record with X. This can be defined more precisely by the expression:

$$\text{confidence}(X \rightarrow Y) = \frac{support(X \cup Y)}{support(X)}$$

Lift: the ratio of the observed Support to that which would be expected if X and Y were independent. This can be defined more precisely by the expression:

$$\text{lift}(X \rightarrow Y) = \frac{support(X \cup Y)}{support(X) * support(Y)}$$

These values and the Apriori and Association Rules Mining algorithms are well known within the retail business, for instance for basket analysis, and webpage views analysis. It has not previously been applied or adapted for the medical domain or healthcare industry.

The present invention relates to a modification of this algorithm for the healthcare domain. The general concept underlying this adaptation will now be explained.

For hospital operations, there may be set certain key performance indicators (KPIs) which are required to be met. To meet the KPI, there may be certain subsidiary satisficing conditions which need to be met pertaining to one or more clinical actions (e.g. intervention) or patient conditions. The result for each of these satisficing conditions for each patient or treatment may be stored in a database, along with multiple other factors pertaining to the patient or the treatment in question.

By way of illustration, a given satisficing condition, **T**, may be assumed to have binary value Y(es) or N(o). A value of Yes for the satisficing condition might be represented by the letter *t*, i.e. $\{t = Y | Y \in \textbf{\textit{T}}\}$.

Embodiments of the present invention are aimed at identifying strength of association between co-occurrence of some group or subset of factors included in the dataset, e.g. (A, B), and the attaining of a positive Y condition value, i.e. *t*, for the satisficing condition. This may be understood as seeking to determine the extent to which the following rule is true: (A, B) $\rightarrow$ t (i.e. presence of A and B together entails *t*).

The key definitions in the modified Association Rules Mining and Apriori Algorithm as applied in embodiments of the present invention may accordingly be defined, for the relevant rule (A, B) $\rightarrow$ t, as follows.

Support: how frequently the chosen factors A and B are present together in the same data record of the dataset P, when the satisficing condition is also satisfied for that record, i.e. *t* holds for that record. This can be defined more precisely by the expression:

$$support\big((A,B) \cup t\big) = \frac{|(A,B) \cup t|}{|P|}$$

Confidence: how often the rule (A, B) $\rightarrow$ t has been found to be true, i.e. in how many cases of the factors A and B appearing together in a record of the dataset P, the satisficing condition is also satisfied for that record, i.e. *t* is also true for that record. This can be defined by precisely by the expression:

$$\mathrm{confidence}\big((A, B) \cup t\big) = \frac{support\big((A,B) \cup t\big)}{support\big((A,B)\big)}$$

Lift: the ratio of the observed support of the rule (A, B) → t to that expected if (A, B) and the satisficing condition *t* were independent. This can be defined more precisely by the expression:

$$\mathrm{lift}\big((A, B) \to t\big) = \frac{support\big((A,B) \cup t\big)}{support\big((A,B)\big) * support(t)}$$

Having derived these parameters, it can be determined which factors are associated with a positive result for meeting the KPI satisficing condition, i.e. with t, and also the strength of this association, i.e. and how much impact these factors have on the this condition.

In the above definitions for the modified version of the Apriori Algorithm, P represents the whole given dataset, i.e. the whole patient population, the letter t represents the satisfying of the KPI condition in a given record, and (A, B) represents a bi-factor subset of factors A, B being present together in the same record.

Although a bi-factor set is used for illustrative purposes in the above definitions, the relevant subset of factors may in fact contain any plural number of factors. Hence (A, B) in the above expressions may be replaced for example with (A, B, C) or (A, B, C, D), or more generally (A, B,..., n) where n is an integer less than or equal to the total number of factors in the dataset (though preferably less than the total number of factors in the dataset).

However, it is noted that increasing the number of factors in the subset will in general reduce the number of matching records, leading to a smaller Support value. It may be of advantage therefore in accordance with certain examples to limit the number of factors in each subset so as to maintain some minimum Support value, to ensure that each subset of factors is representative, to some minimum degree, of the complete set of records in the dataset.

Known approaches to analyzing stored clinical data are able to derive the significance of only single factors for the satisfying of a given target condition. However, such an approach is overly simplistic, since in practice, interrelation between multiple factors is often an important underlying driver for meeting or failing certain conditions. Significant correlations and covariations between multiple factors are not at present being taken into account by known methods. As a result, certain key trends may be missed, which may lead to misjudgments by clinicians and medical professionals.

The approach according to embodiments of the present invention provides multi-variate factor mining by determining association between subsets of multiple factors and satisfying of a given condition result. Applied in the healthcare domain, this can lead to improvement in patient outcomes within hospitals and medical centers, since linkage between groups of factors associated with individual patients, such as factors related to medical status or history, or behavior or practices of the patient, and conditions e.g. related to patient or treatment outcome or other requirements related to treatment or consultation, e.g. waiting time, can be determined. This allows the conditions to be more reliably met in future, since the combinations of factors leading to those conditions, or failure in those conditions, can be identified.

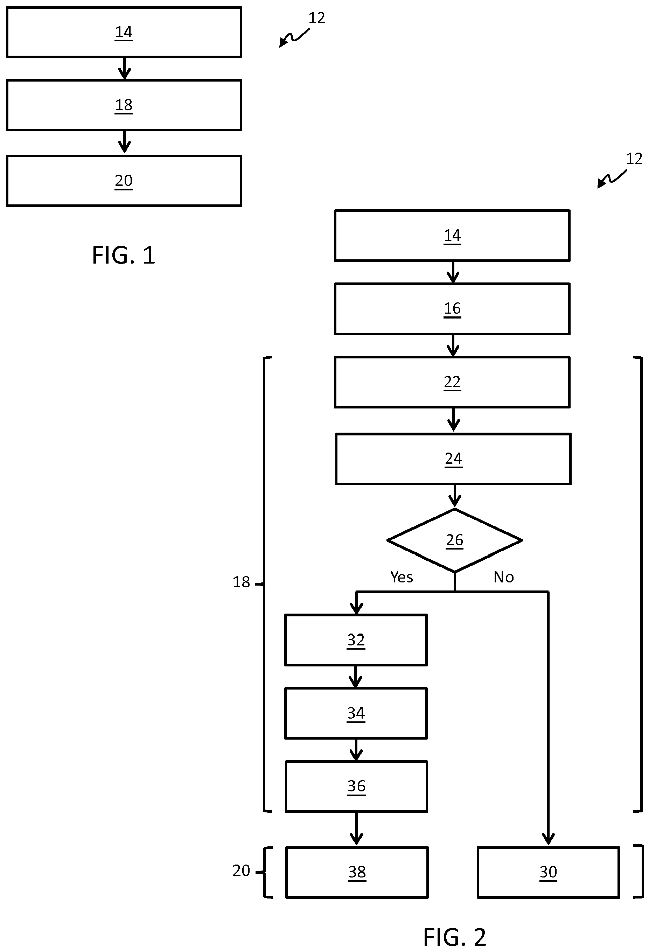

To explain the concept of the invention in more detail, Fig. 1 shows in block diagram form one example method 12 in accordance with one or more embodiments. This example method is for determining factors in a dataset whose co-occurrence lead to satisfying of at least one condition in the healthcare domain. This example method will now be described.

The method 12 comprises first retrieving 14 a dataset, P, the fields of the dataset including a plurality of healthcare factors, and at least one condition result in the healthcare domain, wherein each of the factors and the condition result have binary values.

The dataset may be retrieved for instance form a local or remote memory, for instance from a central server. The dataset may be a database or may be embodied in the form of a database or other data structure. The dataset is composed of a set of data records, each record preferably pertaining to an individual patient. Each record may in some examples pertain to an individual treatment, consultation or other clinical action pertaining to the patient. Each record includes entries for each of a plurality of fields of the dataset. The fields include a plurality of healthcare-related factors, and include a satisfaction result for at least one condition. Each of said plurality of healthcare factors and the satisfaction result take binary values, e.g. Y(es) / N(o).

By way of non-limiting illustration, the healthcare factors might include for instance whether the patient is a smoker, whether the patient is an alcoholic, whether the patient is obese, whether the patient has high blood pressure, whether the patient is diabetic, whether the patient is over 70 or any other healthcare related factor. By way of non-limiting illustration, the condition may pertain for instance to a success measure for patient outcome following intervention, thresholds in patient satisfaction ratings, maximum allowable rates for infection contraction, waiting time thresholds, mortality rates in certain domains, average length of patient stay for certain treatments or clinical domains and many others as will be apparent to the skilled person.

Following retrieval of the dataset P, the method may optionally comprise applying 16 a pre-filtering step adapted to reduce a number of records in the dataset having a No value for the condition result. This may comprise for instance filtering the records of the dataset in a manner configured to select those records having a positive (Yes) value for the condition result and/or to reject those record having a negative (No) value for the condition result. This step improves processing efficiency and speed for the method. This is because the method aims at identifying associations between groups of factors and the positive satisfaction of one or more condition results. Hence, records containing a negative result are less relevant or useful to the method. Removing at least a portion of these records in advance of the remainder of the method reduces the quantity of data which must be processed (e.g. for which confidence and/or lift values must be determined), and hence improves processing efficiency and speed.

The optional pre-filtering step may comprise use of a Random Forest or Chi Squared test adapted to identify certain of the factors which have significant influence upon the condition outcome. Applying the pre-filtering step reduces but the total number of records in the dataset, P, having a negative (No) value for the condition result, but may not completely eliminate all records having negative (No) values. For example, a dataset which originally contains 68 records having a positive (Yes) value for the condition result and 32 records having a negative (No) value for the condition result might after the pre-filtering step be reduced to having 60 records with a positive (Yes) value for the condition result and 10 records having a negative (No) value. The pre-filtering step hence is adapted to reduce the number of records having a negative (No) value to a greater extent than it reduces the number of records having a positive (Yes) value.

For each of a plurality of plural subsets, (A, B,....,n), of the factors, Confidence, and/or Lift values are calculated 18 using the Apriori Algorithm or Association Rules Mining Algorithm, these having the general form,

$$\text{confidence}(X \cup Y) = \frac{support(X \cup Y)}{support(X)}$$

$$\text{lift}(X \rightarrow Y) = \frac{support(X \cup Y)}{support(X) * support(Y)}$$

wherein co-occurrence in a single record of positive (Yes) values for each of the factors of a given subset is used as X in the Algorithm, and a positive (Yes) value of the condition result in said same record is used as Y in the Algorithm, and where, in general for a given factor or condition $\phi$,

$$support(\phi) = \frac{|\phi|}{|P|}$$

Hence the Confidence, Support and Lift values take the general form defined in the expressions set out previously. Co-occurrence in a single record of positive values for all factors in a given subset is used as X in the expressions. A positive result of the condition in a same subset is used as Y in the expression.

Confidence and/or lift values are calculated in respect of each of a plurality of plural sets of the factors. In this way different numbers and combination of factors can be tested for their strength of association with the positive condition outcome. By testing many different combinations of factors, the particular factors which in combination are most strongly associated with, i.e. most reliably lead to, positive satisfaction of the condition result can be determined.

Based on the calculated Confidence and/or Lift values for the plurality of subsets of the factors, the method further comprises determining 20 which of the factors of the dataset together lead to a positive value for the condition result. This can be achieved in a number of different ways, specific examples of which will be described further below. In a simple example for instance, the factors belonging to the one or more subsets which achieve the highest confidence and/or lift scores are determined to be the factors which lead to a positive value for the condition result. Both confidence and lift values may be determined and taken into account in some examples. In other examples, just one of confidence and lift values may be taken into account. In some cases, which of the parameters (support and confidence) are taken into account may be determined in part based on outcomes for values of one or other of the parameters. Such an example will be described in detail further below.

According to one or more embodiments, the plurality of plural subsets, (A, B,...,n), of the factors may comprise subsets containing different number of factors. By this is meant that certain of the subsets contain different numbers of factors to other of the subsets. This means that the method is not limited by any pre-prepared assumption concerning how many factors tend to lead to a particular condition result.

By testing many different subsets with different numbers and combinations of factors for their degree of association with a given condition result, both the particular number of the attributes, and which of the attributes, that lead to the satisfying of the given condition may be determined.

In advantageous examples, the method may be applied for determining attributes which lead to the satisfying of a key performance indicator (KPI) for a healthcare provider to which data in the dataset relates. This key performance indicator may encompass or require the satisfaction of a plurality of target conditions, each of which is included as a field in the dataset. The key performance indicators may by way of non-limiting example pertain to any of the following: attaining a defined metric of success in patient outcomes, thresholds in patient satisfaction ratings, maximum allowable rates for infection contraction, waiting time thresholds, mortality rates in certain domains, average length of patient stay for certain treatments or clinical domains and many others.

According to this or any other example, the fields of the dataset may advantageously encompass a plurality of condition results in the healthcare domain. The method may involve performing each of said steps of: calculating confidence and/or lift values, and determining which of the plurality of subsets of factors leads to a positive value for the condition result in respect of each of the plurality of condition results. In the case that the method includes the optional pre-filtering step performed following retrieval of the dataset, P, and in advance of calculating the confidence and/or lift values, then said pre-filtering step may also be performed in respect of each of the plurality of condition results.

In this way associations between subsets of factors in the dataset and multiple different conditions can be separately identified.

For example, the KPI target T may encompass the meeting of more than one condition, e.g. (Y1, Y2...Ym). After the applying the algorithm in respect of each of these conditions, certain factors or attributes (X1, X2, X5) may be determined to lead to Y1, some factors or attributes (X1, X2, X6, X8, X9) may be determined to lead to Y2,...some factors or attributes (X1, X2, X10, X11) may be determined to lead to Ym. Finally, the attributes (X1, X2) that are found to contribute to the satisfying of each and every one of the conditionsY1, Y2... Ym are determined to be the set of attributes which (most reliably) lead to the meeting of the target T.

Embodiments of the invention permit detection of particular combinations of factors whose co-occurrence together reliably tend to lead to satisfaction of some condition. This hence takes into account that it is often not a single factor which leads to a particular condition result, but rather the interaction and combination of multiple factors. Prior art approaches in general are based on ranking single factors in terms of their frequency of association with a given condition result. The highest ranking factors are then selected as those which lead to the condition result, and hence those which should be focused on for improvement in frequency of attaining the condition result.

However, this can give a skewed result, because some factors may be ordered relatively low in such a ranking, but which, when present in combination with certain other factors, lead to a very high likelihood of the condition being satisfied. It is of value to identify such factors since by focusing on these, in combination with those others, the condition might more reliably be satisfied than by focusing on the high ranking factors alone.

In general, the output of the method in use is a set of factors which together are determined to lead to satisfying of a particular condition, these being selected from the plurality of subsets of factors which are tested. This condition may be a key performance indicator for instance, or the condition may be one of multiple conditions encompassed by a key performance indictor.

In either case, this information allows identification of particular factors which should be focused on or improved in order to increase rates of satisfaction of the condition or performance indicator.

This confers a clear advantage for patients of improving certain factors related to their treatment or likely outcome, for example improving patient mortality rate (improving 5-year survival post treatment for instance), or improving the experience of the patient in the hospital.

For the hospital or healthcare center, there is a clear advantage of enabling efficient improvements in the treatment plan or diagnosis accuracy and in the management of patient outcome and patient experience.

For example, in the case of a condition target which represents a key performance indicator (KPI), by analyzing the co-occurrence of attributes that lead to the KPI, the hospital may take actions to intervene in respect of at least one of these attributes so as to reduce or increase (as appropriate) the possibility that the attributes will co-occur in the future.

By way of example, the attributes (Smoker & Alcoholic) and (Smoker & Obesity) may be determined as leading to Mortality. In order to reduce the mortality rate, interventions may be planned and provided to any patient who is both a smoker and an alcoholic or any patient who is both a smoker and has obesity. If the patient who is both a smoker and an alcoholic can either stop smoking or stop drinking alcohol, the patient may be more likely to recover. If the patient who is both a smoker and has obesity can either stop smoking or increase exercise levels, the patient may again be more likely to recover.

As discussed above, the embodiments of the invention hence differ from known methods in which all factors are ranked from the most relevant to the least relevant. Intervening only in respect of factors ranking at the top of such a list may not achieve maximally beneficial results, since a factor X1, ranking further toward the bottom, may have lower relevance by itself, but may, in combination with one or more other factors be found to very reliably lead to satisfying of a given condition Y. The hospital hence should also intervene in the factor X1, which may for instance be easier to address than some other contributing factors. Lack of information concerning the correlation between two or more factors may lead to missing key opportunities to effectively improve meeting of conditions and performance targets. This can increase risk for patients. By understanding association between the co-occurrence of certain factors and the attainment of a given condition or target, a hospital may put more resources into addressing those factors and thereby improve rates of attainment of the KPI target in a more efficient way.

As discussed above, the method involves determining confidence and/or lift values of the Apriori Algorithm in respect of each of the plurality of subsets of factors. These values may be determined sequentially in some examples, with the outcome for one (e.g. in respect of all of the subsets) informing whether or not the other is to be determined.

This can reduce processing demand and increase processing speed since in at least some cases, only the confidence values need to be determined and not the lift values, or vice versa.

One advantageous example method 12, in accordance with one or more embodiments, which applies this approach, will now be outlined with reference to Fig. 2.

Steps of this example corresponding to those of the example of Fig. 1 are labeled with similar reference numerals. In particular, the first step of the method is unchanged compared to the method of Fig. 1.

Hence, the method 12 first comprises retrieving 14 a dataset, P, the fields of the dataset including a plurality of healthcare factors, and at least one condition result in the healthcare domain, wherein each of the factors and the condition result have binary values.

In this example, following retrieval of the dataset P, the method comprises a pre-filtering step 16 adapted to reduce a number of records in the dataset having a No value for the condition result..

Following this, the method comprises first calculating 22 Confidence values in respect of each of said plurality of subsets of factors, using the Apriori Algorithm or Association Rules Mining Algorithm. As in the example of Fig. 1, these have the general form,

$$\text{confidence}(X \cup Y) = \frac{support(X \cup Y)}{support(X)}$$

wherein the co-occurrence in a single record of positive values for each of the factors in a given subset is used as X in the Algorithm, and a positive value of the condition result is used as Y in the Algorithm. The support value again takes the following general definition, for a given factor or condition $\phi$:

$$support(\phi) = \frac{|\phi|}{|P|}$$

Following calculation of the Confidence values, the method comprises identifying 24 a maximum of the calculated Confidence values, i.e. identifying a largest of the calculated confidence values.

The method then comprises determining 26 whether the maximum value has been calculated/returned for more than one of said subsets, i.e. if more that one of the subsets shares this maximum Confidence value.

If not ("No"), then the factors of the single subset for which the maximum confidence value has been returned are determined 30 to be the factors which lead to a positive value of said condition result. These factors are provided as, or form, an output of the method.

If, by contrast, more than one of the subsets does share the maximum Confidence value, then the method further comprises determining 32 Lift values in respect of each of said subsets for which the maximum Confidence value is determined (each of the subsets sharing the maximum Confidence value). Hence, Lift values are only determined in the case that more than one of the subsets of factors shares the maximum determined confidence value.

Once the Lift values are accordingly calculated, the method comprises identifying 34 a maximum of the calculated Lift values, i.e. identifying a largest of the calculated Lift values.

Following this, the method comprises identifying 36 which one or more of the subsets of factors said maximum Lift value has been calculated for.

Following this, the method then comprises determining 38 the factors of said identified subset(s) to be the factors which together lead to a positive value of said condition result. These may then be provided, or may form, an output of the method.

According to certain examples, only factors shared by each and every one of the identified subset(s) are determined as those which together lead to a positive value of said condition result.

Optionally, the method may comprise an additional step, after determining 22 the Confidence values, and in advance of identifying 24 a maximum of the confidence values, of applying a minimum threshold to the calculated confidence values, such that a maximum is identified only among the calculated confidence values exceeding said threshold.

This effectively filters the confidence values, to reduce the total quantity of results which need to be compared in order to determine the maximum value.

To further illustrate the invention two specific examples of application of a method in accordance with one or more embodiments will now be outlined in detail.

Table 1 shows a hypothetical dataset for a hospital, presented by way of illustration. This dataset includes fields pertaining to three different healthcare-related factors: whether the patient is a smoker, whether the patient is an alcoholic, and whether the patient is obese. The table includes one field pertaining to a target condition, the condition being the meeting of a 30-day Mortality rate. This by way of example is assumed to be a key performance indicator (KPI) for the hospital. A positive value (=Y) is sought as the satisficing condition for meeting this condition. The support, confidence, and lift values are calculated in respect of each combination of two factors and the condition, and these are presented beneath the dataset. Only combinations of two factors are considered due to the limited total number of factors (only three) included in this simple illustrative example.

**Table 1**

| Smoker | Alcoholic | Obesity | 30-day Mortality |
|--------|-----------|---------|------------------|
| Y | Y | Y | Y |
| Y | Y | N | Y |
| Y | N | N | N |
| N | Y | Y | N |
| Y | Y | N | Y |
| N | Y | Y | Y |
| Y | N | N | N |

Support(Smoker & Mortality) = 3/7
Support(Alcoholic & Mortality) = 4/7
Support(Obesity & Mortality) = 2/7
Support((Smoker & Alcoholic) & Mortality) = 3/7
Support((Alcoholic & Obesity) & Mortality) = 2/7
Support((Smoker & Obesity) & Mortality) = 1/7
Confidence(Smoker -> Mortality) = (3/7)/(5/7) = 3/5
Confidence(Alcoholic -> Mortality) = (4/7)/(5/7) = 4/5
Confidence(Obesity -> Mortality) = (2/7)/(3/7) = 2/3
Confidence((Smoker & Alcoholic) -> Mortality) = (3/7)/(3/7) = 3/3 = **1**
Confidence((Alcoholic & Obesity) -> Mortality) = (2/7)/(3/7) = 2/3
Confidence((Smoker & Obesity) -> Mortality) = (1/7)/(1/7) = **1**
Lift(Smoker -> Mortality) = (3/7)/((5/7)*(4/7)) = 1.05
Lift(Alcoholic -> Mortality) = (4/7)/((5/7)*(4/7)) = 1.4
Lift(Obesity -> Mortality) = (2/7)/((3/7)*(4/7)) =1.167
Lift((Smoker & Alcoholic) -> Mortality) = (3/7)/((3/7)*(4/7)) = **1.75**
Lift((Alcoholic & Obesity) -> Mortality) = (2/7)/((2/7)*(4/7)) = 1.75
Lift((Smoker & Obesity) -> Mortality) = (1/7)/((1/7)*(4/7)) = **1.75**
The results show that the subset of factors (Smoker & Alcoholic) and (Smoker & Obesity) lead to Mortality with the highest confidence (of 1, i.e. 100%). These are highlighted in bold. These combinations of factors each have a lift of 1.75 times higher than in the case that the factors were independent. Since these subsets of factors achieved the highest confidence values, it might be concluded that the factors of theses subsets most reliably lead to satisfying of the condition.

Although in the above example, Lift values were determined in respect of every combination of factors, in other examples, Lift values might instead be determined only in respect of (Smoker & Alcoholic) and (Smoker & Obesity) as these were determined to share a maximum of the calculated Confidence values. Since both of these also shared the maximum calculated Lift value, the factors of both may be output as the factors which lead most reliably to satisfying of the condition.

A further illustrative example will now be described below. Table 2 shows a further hypothetical dataset for a hospital. This dataset again includes fields pertaining to three different healthcare-related factors: whether the patient has hypertension, whether the patient has coronary heart disease, and whether room turn-around time for the patient was equal to or greater than 10 days. The table includes one field pertaining to a target condition, the condition being a patient waiting time of greater than or equal to 2 days. Confidence and lift values will be calculated in respect of a positive value (Y) for this condition, so that the hospital can aim to avoid those combinations of factors which lead to positive satisfying of this condition (the aim is to reduce waiting times). This by way of example may be a key performance indicator (KPI) for the hospital.

The support, confidence, and lift values are calculated in respect of each combination of two factors, in respect of meeting the condition, and these are presented beneath the dataset. Again, only combinations of two factors are considered due to the limited total number of factors (only three) included in this simple illustrative example.

**Table 2**

| Hypertension | Coronary Heart Disease | Room turn-around time >= 10 days | Patient waiting time >=2 days |
|---|---|---|---|
| Y | Y | Y | Y |
| Y | Y | N | Y |
| Y | N | N | N |
| N | Y | Y | N |
| Y | N | Y | Y |
| N | Y | Y | N |
| Y | Y | N | Y |

As noted, the target KPI in this case is the patients waiting time > = 2 days (to producers).

Support (Hypertension &Patients waiting time >=2 days) = 4/7
Support (Coronary Heart Disease & Patients waiting time >= 2 days) = 3/7
Support (Room Turn Around Time >=10 days & Patients waiting time >= 2 days) = 2/7
Support ((Hypertension & Coronary Heart Disease) &Patients waiting time >= 2 days) = 3/7
Support ((Hypertension & Room Turn Around Time) &Patients waiting time >= 2 days) = 2/7
Support ((Coronary Heart Disease & Room Turn Around Time >=10 days) &Patients waiting time >=2 days) = 1/7

Confidence (Hypertension → Patients waiting time >= 2 days) = (4/7)/(5/7) =4/5
Confidence(Coronary Heart Disease → Patients waiting time >= 2 days) = (3/7)/(5/7) = 3/5
Confidence (Room Turn Around Time >= 10 days → Patients waiting time >=2 days) = (2/7)/(4/7) =1/2
Confidence ((Hypertension & Coronary Heart Disease) → Patients waiting time >= 2 days) = (3/7)/(3/7)=**1**
Confidence ((Hypertension & Room Turn Around Time) → Patients waiting time >= 2 days) = (2/7)/(2/7)=**1**
Confidence ((Coronary Heart Disease & Room Turn Around Time) → Patients waiting time >= 2 days) = (1/7)/(3/7) = 1/3

Lift (Hypertension & Patients waiting time >= 2 days) = (4/7)/ ((5/7) * (4/7) = 7/5 = 1.4
Lift (Coronary Heart Disease & Patients waiting time >= 2 days) = (3/7)/ ((5/7) * (4/7)) =21/20 = 1.05
Lift (Room Turn Around Time >=10 days & Patients waiting time >= 2 days) = (2/7) / ((4/7) * (4/7)) = 7/8 = 0.875
Lift ((Hypertension & Coronary Heart Disease) & Patients waiting time >= 2 days)) = (3/7) / ((3/7) * (4/7)) = 7/4 = **1.75**
Lift ((Hypertension& Room Turn Around Time >=10 days) & Patients waiting time >= 2 days) = (2/7)/ ((2/7) * (4/7)) = 7/4 = **1.75**

The results show that the two subsets (Hypertension & Coronary Heart Disease) and (Hypertension & Room Turn Around Time >= 10 days) achieve a joint or shared highest confidence value, which in this case is 100% (these are shown in bold).

Lift values were hence calculated in respect of these two subsets of factors. Both achieved the same Lift value of 1.75, meaning a lift of 1.75 times higher than factors which are independent. Thus, it might be concluded that more attention should be paid to any patients who have both positive hypertension & coronary heart disease or both positive hypertension & room turn-around time >= 10 days, since these patients are likely to experience longer waiting times. Monitoring waiting times for these patients specifically hence may allow long wait times to be remedied, enabling the key performance indicator of not exceeding 2 days waiting time for each patient to be better met.

A further aspect of the invention provides a computer program product comprising a non-transitory computer readable medium, the computer readable medium having computer readable code embodied therein, the computer readable code being configured such that, on execution by a suitable computer or processor, the computer or processor is caused to performed a method in accordance with any of the examples or embodiments outlined above or in accordance with any claim of this application.

Examples in accordance with a further aspect of this invention provide a system comprising:

a memory comprising instruction data representing a set of instructions; and
a processor configured to communicate with the memory and to execute the set of instructions, wherein the set of instructions, when executed by the processor, cause the processor to perform the steps of a method in accordance with any of the examples or embodiments outlined or described above, or as set out in any claim of this application.

Specifically, instructions, when executed by the processor, cause the processor at least to:

retrieve a dataset, P, the fields of the dataset including a plurality of healthcare factors, and at least one condition result in the healthcare domain, wherein each of the factors and the condition result have binary values;
calculate, for each of a plurality of plural subsets, (A, B,....,n), of the factors, Confidence, and/or Lift values using the Apriori Algorithm, these having the form,

$$\text{confidence}(X \cup Y) = \frac{support(X \cup Y)}{support(X)}$$

$$\text{lift}(X \rightarrow Y) = \frac{support(X \cup Y)}{support(X) * support(Y)}$$

wherein co-occurrence in a single record of positive values for each of the factors in a given subset is used as X in the Algorithm, and a positive value of the condition result in the same subset is used as Y in the Algorithm, and where, for a given factor or condition $\phi$,

$$support(\phi) = \frac{|\phi|}{|P|}$$

and;
determine factors of the dataset which lead to a positive value for the condition result based on the calculated Confidence and/or Lift values for the plurality of subsets of the factors.

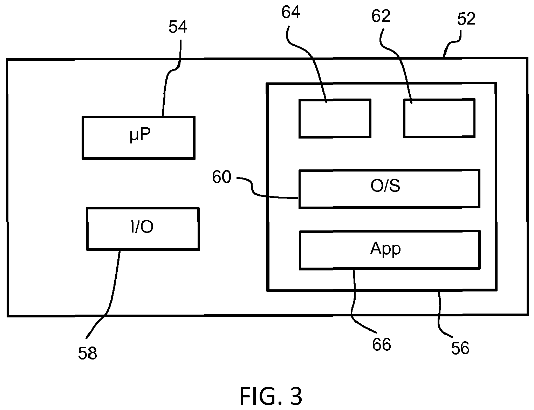

By way of example, Fig. 3 illustrates an example of a computer 52 for implementing the system described above.
The computer 52 includes, but is not limited to, PCs, workstations, laptops, PDAs, palm devices, servers, storages, and the like. Generally, in terms of hardware architecture, the computer 52 may include one or more processors 54, memory 56, and one or more I/O devices 58 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.
The processor 54 is a hardware device for executing software that can be stored in the memory 56. The processor 54 can be virtually any custom made or commercially available processor, a central processing unit (CPU), a digital signal processor (DSP), or an auxiliary processor among several processors associated with the computer 52, and the processor 54 may be a semiconductor based microprocessor (in the form of a microchip) or a microprocessor.
The memory 56 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 56 may incorporate electronic, magnetic, optical, and/or other types of storage media. Note that the memory 56 can have a distributed architecture, where various components are situated remote from one another, but can be accessed by the processor 54.
The software in the memory 56 may include one or more separate programs, each of which comprises an ordered listing of executable instructions for implementing logical functions. The software in the memory 56 includes a suitable operating system (O/S) 60, compiler 62, source code 64, and one or more applications 66 in accordance with exemplary embodiments.
The application 66 comprises numerous functional components such as computational units, logic, functional units, processes, operations, virtual entities, and/or modules.

The operating system 60 controls the execution of computer programs, and provides scheduling, input-output control, file and data management, memory management, and communication control and related services.

Application 66 may be a source program, executable program (object code), script, or any other entity comprising a set of instructions to be performed. When a source program, then the program is usually translated via a compiler (such as the compiler 62), assembler, interpreter, or the like, which may or may not be included within the memory 52, so as to operate properly in connection with the operating system 60. Furthermore, the application 66 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 58 may include input devices such as, for example but not limited to, a mouse, keyboard, scanner, microphone, camera, etc. Furthermore, the I/O devices 58 may also include output devices, for example but not limited to a printer, display, etc. Finally, the I/O devices 58 may further include devices that communicate both inputs and outputs, for instance but not limited to, a network interface controller (NIC) or modulator/demodulator (for accessing remote devices, other files, devices, systems, or a network), a radio frequency (RF) or other transceiver, a telephonic interface, a bridge, a router, etc. The I/O devices 58 also include components for communicating over various networks, such as the Internet or intranet.

When the computer 52 is in operation, the processor 54 is configured to execute software stored within the memory 56, to communicate data to and from the memory 56, and to generally control operations of the computer 52 pursuant to the software. The application 66 and the operating system 60 are read, in whole or in part, by the processor 54, perhaps buffered within the processor 54, and then executed.

When the application 66 is implemented in software it should be noted that the application 66 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A method (12) for determining factors in a dataset whose co-occurrence lead to satisfying of at least one condition in the healthcare domain, the method comprising:

retrieving (14) a dataset, P, the fields of the dataset including a plurality of healthcare factors, and at least one condition result in the healthcare domain, wherein each of the factors and the condition result have binary, Yes/No, values;
calculating (18), for each of a plurality of plural subsets, (A, B,.....,n), of the factors, Confidence, and/or Lift values using the Apriori Algorithm, these having the form,

$$\text{Confidence}(X \cup Y) = \frac{support(X \cup Y)}{support(X)}$$

$$\text{Lift}(X \rightarrow Y) = \frac{support(X \cup Y)}{support(X) * support(Y)}$$

wherein co-occurrence in a single record of Yes values for each of the factors of a given subset is used as X in the Algorithm, and a Yes value of the condition result in said same record is used as Y in the Algorithm, and where, in general for a given factor or condition $\phi$,

$$support(\phi) = \frac{|\phi|}{|P|}$$

and;
determining (20) factors of the dataset which together lead to a Yes value for the condition result based on the calculated Confidence and/or Lift values for the plurality of subsets of factors.

2. A method (12) as claimed in claim 1, wherein the condition is a Performance Indicator for a healthcare provider to which data in the dataset relates.

3. A method (12) as claimed in claim 1, wherein the method further comprises, following retrieval (14) of the dataset, P, and in advance of calculating (18) said Confidence and/or Lift values, applying a pre-filtering step (16) adapted to reduce a number of records in the dataset having a No value for the condition result.

4. A method as claimed in claim 1, wherein
the fields of the dataset include a plurality of condition results in the healthcare domain, and
wherein said steps of: calculating confidence and/or lift values, and determining which of the plurality of subsets of factors leads to a Yes value for the condition result are performed in respect of each of the plurality of condition results.

5. A method (12) as claimed in claim 4, wherein the method is for determining factors in the dataset whose co-occurrence leads to satisfying of a performance Target in a healthcare domain, satisfying of said Target involving satisfying of said plurality of condition results.

6. A method (12) as claimed in claim 1, wherein the plurality of plural subsets, (A, B,...,n), of the factors comprises subsets containing different number of factors.

7. A method (12) as claimed in claim 1 wherein the method comprises:

first calculating (22) Confidence values in respect of each of said plurality of subsets of factors,
identifying (24) a maximum of the calculated Confidence values; and
determining (32) Lift values only in the case that more than one of said subsets shares said maximum confidence value, the lift values being determined only in respect of said subsets sharing the maximum confidence value.

8. A method (12) as claimed in claim 7, wherein in the case that said maximum of the confidence values is returned for only one of said subsets of factors, the factors of that subset are determined (30) as the factors which lead to a Yes value of said condition result.

9. A method (12) as claimed in claim 7, wherein in the case that said Lift values are determined (32), the method further comprises:

identifying (34) a maximum of the calculated Lift values;
identifying (36) which of the subsets of factors said maximum Lift value has been calculated for; and
determining (38) the factors of said identified subset(s) to be the factors which lead to a Yes value of said condition result.

10. A method (12) as claimed in claim 7, comprising applying a minimum threshold to said calculated confidence values, such that a maximum is identified only among the calculated confidence values exceeding said threshold.

11. A method (12) as claimed in claim 1, wherein the at least one condition includes one or more of:

the satisfying of a minimum survival period of a patient following a medical procedure; and
compliance with a maximum patient waiting time threshold.

12. A computer program product comprising a non-transitory computer readable medium, the computer readable medium having computer readable code embodied therein, the computer readable code being configured such that, on execution by a suitable computer or processor, the computer or processor is caused to performed the method of any of claims 1-11.

13. A system comprising:

a memory (56) comprising instruction data representing a set of instructions; and
a processor (54) configured to communicate with the memory and to execute the set of instructions, wherein the set of instructions, when executed by the processor, cause the processor to:

retrieve (14) a dataset, P, the fields of the dataset including a plurality of healthcare factors, and at least one condition result in the healthcare domain, wherein each of the factors and the condition result have binary values;
calculate (18), for each of a plurality of plural subsets, (A, B,....,n), of the factors, Confidence, and/or Lift values using the Apriori Algorithm, these having the form,

$$\text{confidence}(X \cup Y) = \frac{support(X \cup Y)}{support(X)}$$

$$\text{lift}(X \rightarrow Y) = \frac{support(X \cup Y)}{support(X) * support(Y)}$$

wherein co-occurrence in a single record of Yes values for each of the factors of a given subset is used as X in the Algorithm, and a Yes value of the condition result in said same record is used as Y in the Algorithm, and where, in general for a given factor or condition $\phi$,

$$support(\phi) = \frac{|\phi|}{|P|}$$

and;
determine (20) factors of the dataset which lead to a Yes value for the condition result based on the calculated Confidence and/or Lift values for the plurality of subsets of factors.

14. A system as claimed in claim 13, wherein said set of instructions, when executed by the processor, cause the processor to:

first calculate (22) Confidence values in respect of each of said plurality of subsets of factors,
identify (24) a maximum of the calculated Confidence values; and
determine (32) Lift values only in the case that more than one of said subsets shares said maximum confidence value, the lift values being determined only in respect of said subsets sharing the maximum confidence value.

15. A system as claimed in claim 14, wherein in the case that said Lift values are determined (32), the instructions, when executed by the processor, further cause the processor to:

identify (34) a maximum of the calculated Lift values;
identify (36) which of the subsets of factors said maximum Lift value has been calculated for; and
determine (38) the factors of said identified subset(s) to be the factors which lead to a Yes value of said condition result.

12

| 14 |
| 18 |
| 20 |

FIG. 1

12

| 14 |
| 16 |
| 22 |
| 24 |

26

Yes          No

18

| 32 |
| 34 |
| 36 |

20

| 38 |          | 30 |

FIG. 2

FIG. 3

Europäisches
Patentamt

European
Patent Office

Office européen
des brevets

## EUROPEAN SEARCH REPORT

Application Number

EP 18 20 5425

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 542 532 B1 (MCNAIR DOUGLAS S [US] ET AL) 10 January 2017 (2017-01-10)<br>* figures 1A-6 *<br>* column 1, line 14 - column 3, line 58 *<br>* column 4, line 21 - column 5, line 28 *<br>* column 6, line 19 - column 10, line 7 *<br>* column 12, line 42 - line 67 * | 1,12,13 | INV.<br>G16H40/20<br>G16H50/70<br><br>ADD.<br>G06N5/02 |
| X | US 2012/209625 A1 (ARMSTRONG JOHN M [US] ET AL) 16 August 2012 (2012-08-16)<br>* page 1, paragraph 0002 - page 2, paragraph 0010 *<br>* page 3, paragraph 0026 - paragraph 0032 *<br>* page 5, paragraph 0041 - paragraph 0051; figures 1,2,4A * | 1-15 | |
| X | NEHA SHARMA ET AL: "Early Detection and Prevention of Oral Cancer: Association Rule Mining on Investigations", WSEAS TRANSACTIONS ON COMPUTERS, vol. 13, 31 December 2014 (2014-12-31), pages 1-8, XP055577543, GR ISSN: 1109-2750<br>* the whole document, in particular sections "1 Introduction" on pages 1-2, and "4 Association Rule Mining" * | 1-15 | |

-----

-----

-----

-/--

TECHNICAL FIELDS
SEARCHED (IPC)

G16H
G06N

The present search report has been drawn up for all claims

| Place of search | Date of completion of the search | Examiner |
|---|---|---|
| Munich | 10 April 2019 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................
& : member of the same patent family, corresponding document

EPO FORM 1503 03.82 (P04C01)

EP 3 651 159 A1

# EUROPEAN SEARCH REPORT

**Application Number**

EP 18 20 5425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | THITITHAMAWAT SAHA ET AL: "Association rules to analyze hospital resources with mortality rates", 2018 5TH INTERNATIONAL CONFERENCE ON BUSINESS AND INDUSTRIAL RESEARCH (ICBIR), IEEE, 17 May 2018 (2018-05-17), pages 51-56, XP033361832, DOI: 10.1109/ICBIR.2018.8391165 [retrieved on 2018-06-20] * the whole document, in particular sections "I. introduction" on page 51, "II. Background" on pages 51-52, especially its subsection "B. Apriori Algorithm", "IV. Methodology" on pages 53-54, in particular its subsection, "B. Association Rule Mining" on page 54, and "V. Result" on pages 54-55 * | 1-15 | |
| X | FRAN?OIS S?VERAC ET AL: "Non-redundant association rules between diseases and medications: an automated method for knowledge base construction", BMC MEDICAL INFORMATICS AND DECISION MAKING, BIOMED CENTRAL, LONDON, GB, vol. 15, no. 1, 15 April 2015 (2015-04-15), page 29, XP021221251, ISSN: 1472-6947, DOI: 10.1186/S12911-015-0151-9 * the whole document, in particular abstract and section "Methods" on pages 2-4 * | 1-15 | TECHNICAL FIELDS SEARCHED (IPC) |

-/--

The present search report has been drawn up for all claims

| Place of search | Date of completion of the search | Examiner |
|---|---|---|
| Munich | 10 April 2019 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 18 20 5425

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Anonymous: "Association rule learning - Wikipedia", , 26 October 2018 (2018-10-26), XP055577432, Retrieved from the Internet: URL:https://en.wikipedia.org/w/index.php?title=Association_rule_learning&oldid=865826483 [retrieved on 2019-04-04] * the whole document * ----- | 1-15 | |
| A | ALTAF WASIF ET AL: "Applications of association rule mining in health informatics: a survey", ARTIFICIAL INTELLIGENCE REVIEW, SPRINGER NETHERLANDS, NL, vol. 47, no. 3, 9 May 2016 (2016-05-09), pages 313-340, XP036154841, ISSN: 0269-2821, DOI: 10.1007/S10462-016-9483-9 [retrieved on 2016-05-09] * the whole document * ----- | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 10 April 2019 | Sasa Bastinos, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT
## ON EUROPEAN PATENT APPLICATION NO.

EP 18 20 5425

This annex lists the patent family members relating to the patent documents cited in the above-mentioned European search report.
The members are as contained in the European Patent Office EDP file on
The European Patent Office is in no way liable for these particulars which are merely given for the purpose of information.

10-04-2019

| Patent document cited in search report | | | Publication date | Patent family member(s) | Publication date |
|---|---|---|---|---|---|
| US 9542532 | B1 | | 10-01-2017 | NONE | |
| US 2012209625 | A1 | | 16-08-2012 | AU 2010239214 A1 | 15-12-2011 |
| | | | | BR PI1015255 A2 | 03-05-2016 |
| | | | | CN 102483818 A | 30-05-2012 |
| | | | | EP 2422305 A1 | 29-02-2012 |
| | | | | JP 2012524945 A | 18-10-2012 |
| | | | | KR 20120049180 A | 16-05-2012 |
| | | | | SG 175300 A1 | 28-11-2011 |
| | | | | US 2012209625 A1 | 16-08-2012 |
| | | | | WO 2010124016 A1 | 28-10-2010 |

EPO FORM P0459

For more details about this annex : see Official Journal of the European Patent Office, No. 12/82